# EUROPÄISCHE PATENTSCHRIFT

(11) **EP 3 692 918 B1**
(45) Veröffentlichungstag und Bekanntmachung des Hinweises auf die Patenterteilung: **19.05.2021**
(21) Anmeldenummer: 19156195.0
(22) Anmeldetag: 08.02.2019
(51) Int. Cl.: A61B 6/00

(54) **LERNBASIERTE KORREKTUR VON RASTERARTEFAKTEN IN DER RÖNTGENBILDGEBUNG**
LEARNING-BASED CORRECTION OF RASTER ARTEFACTS IN X-RAY IMAGING
CORRECTION À BASE D'APPRENTISSAGE DES ARTEFACTS TRAMES DANS L'IMAGERIE PAR RAYONS X

(43) Veröffentlichungstag der Anmeldung: 12.08.2020
(73) Patentinhaber: Siemens Healthcare GmbH, 91052 Erlangen (DE)
(72) Erfinder: Stowasser, Boris, 91056 Erlangen (DE); Bernhardt, Philipp, 91301 Forchheim (DE)

(56) Entgegenhaltungen:
- EP-A1- 1 696 366
- DE-A1-102011 080 279
- JP-A- 2018 068 747
- US-A1- 2018 078 221

## Beschreibung

Die vorliegende Erfindung betrifft ein Verfahren zum Betreiben eines Röntgensystems, das einen Detektor und im Strahlengang vor dem Detektor ein Streustrahlenraster aufweist. Darüber hinaus betrifft die vorliegende Erfindung ein computerimplementiertes Verfahren zum Erzeugen von Objektbilddaten sowie ein (C-Bogen-)Röntgensystem mit einem Detektor und einem im Strahlengang vor dem Detektor befindlichen Streustrahlenraster.

Bei der Röntgenbildgebung entstehen an dem zu untersuchenden Objekt in der Regel Streustrahlen. Während die eigentliche Nutzstrahlung von der Röntgenröhre unmittelbar auf den Detektor beziehungsweise Bildträger gerichtet ist, ist die Streustrahlung nicht gerichtet beziehungsweise besitzt eine davon abweichende Richtung. Die Streustrahlung verursacht am Detektor häufig eine gleichmäßige Dosisverteilung. Mit steigender Energie der Röntgenstrahlung nimmt die Streustrahlung in der Regel ab. Der Anteil der Streustrahlung erhöht sich mit zunehmender Objektdicke. Die Bildqualität und insbesondere das Signal-zu-Rausch-Verhältnis verringern sich durch die Streustrahlung.

Eine Reduzierung der Streustrahlung wird im Allgemeinen durch ein fokussierendes Streustrahlenraster erreicht, welches vor dem Detektor angeordnet ist. Das Streustrahlenraster reduziert den Einfall der Streustrahlung, wodurch der Kontrast des Röntgenbilds erhöht wird. Ein derartiges Streustrahlenraster ist in der Regel aus dünnen Bleilamellen aufgebaut. Zwischen den absorbierenden Bleilamellen befinden sich meist durchlässige Abstandshalter aus Aluminium oder Zellulose. Die Lamellen stehen parallel zur Strahlung, sodass die gerichtete, gewünschte Strahlung durch die Lamellen hindurchdringt, während die Streustrahlung absorbiert wird.

Neuere Entwicklungen verwenden z.B. die Technik des Lasersinterns, um Streustrahlenraster aus z.B. Wolfram mit einem sehr guten Schachtverhältnis herzustellen. Dabei wird unter dem Schachtverhältnis das Verhältnis von Spaltbreite zu Spalthöhe im Raster verstanden. Dieses Verhältnis beträgt herkömmlich oft 1:10.

Ein Problem, das bei der Verwendung eines Streustrahlenrasters entsteht, ist die Abbildung der Rasterstruktur im Bild. Dies bedeutet, dass die abgebildete Rasterstruktur die eigentliche Bildinformation überlagert. Ohne eine Korrektur dieser Rasterstruktur ist der Gebrauch dieses Rasters mit den sehr guten Schachtverhältnissen nicht möglich.

Bislang wird dieses Problem dadurch gelöst, dass man versucht, das Röntgensystem entsprechend zu kalibrieren. Dies bedeutet, dass entsprechende Aufnahmen mit dem Streustrahlenraster, aber ohne Untersuchungsobjekt, gewonnen werden und die Abbildungen des Rasters bei den Aufnahmen mit Untersuchungsobjekt entsprechend subtrahiert werden. Auf diese Weise bleiben die Objektdaten und insbesondere die anatomischen Strukturen auf den Bildern erhalten, während die Rasterstrukturen reduziert beziehungsweise sogar eliminiert werden.

Trotz derartiger Kalibrierungen werden in bestimmten Positionen z.B. des C-Bogens die Rasterstrukturen wieder sichtbar. Dies liegt daran, dass ein C-Bogen nicht unendlich steif ist. Vielmehr verbiegt und/oder verwindet sich der C-Bogen aufgrund des Eigengewichts in Abhängigkeit von der jeweiligen Position im dreidimensionalen Raum. Dies bedeutet, dass in gewissen Positionen des C-Bogens die Rasterstruktur nach dieser subtraktiven Kalibration nicht sichtbar ist, während in anderen Positionen die Rasterstruktur durchaus sichtbar ist. Unter Umständen verwindet oder verbiegt sich der C-Bogen derart, dass sich die Ausrichtung von Röntgenquelle und Röntgendetektor in machen Positionen des C-Bogens um Bruchteile eines Grads verändern. Dies genügt, um die entsprechender Rasterstruktur im Bild nicht ausreichend eliminieren zu können. Dementsprechend steigt das Signal-zu-Rausch-Verhältnis bzw. sind Rasterartefakte als störende Strukturen erkennbar.

Die Aufgabe der vorliegenden Erfindung besteht somit darin, Voraussetzungen zu schaffen, um in Röntgenbildern Artefakte eines Streustrahlenrasters einfacher eliminieren zu können.

Erfindungsgemäß wird diese Aufgabe gelöst durch ein Verfahren und ein Röntgensystem nach einem der unabhängigen Ansprüche. Vorteilhafte Weiterbildungen der Erfindung ergeben sich aus den abhängigen Ansprüchen.

Entsprechend einem Aspekt der vorliegenden Erfindung ist demnach vorgesehen ein Verfahren zum Trainieren einer Funktion eines Röntgensystems, das eine Positioniereinrichtung, insbesondere einen C-Bogen, mit einem Detektor und im Strahlengang vor dem Detektor ein Streustrahlenraster aufweist, durch durch
- Positionieren des Detektors an einer Vielzahl an verschiedenen Positionen, wobei sich die Positioniereinrichtung verbiegt und/oder verwindet,
- Aufnehmen jeweils mindestens einer Röntgenaufnahme in jeder der Positionen und
- automatisches Lernen von durch das Streustrahlenraster erzeugter Artefakte aus allen Röntgenaufnahmen für die Funktion in einem ersten Lernschritt.

Es soll also eine Funktion eines Röntgensystems trainiert werden, wobei hier unter dem Trainieren auch ein initiales Anlernen des Röntgensystems zu verstehen ist. Das Röntgensystem ist nämlich in der Lage, das eigene Verhalten insbesondere hinsichtlich seiner Geometrie selbst zu lernen.

Bei dem Röntgensystem handelt es sich vorzugsweise um ein C-Bogen-Röntgensystem oder ein System mit Roboterarmen. Das Röntgensystem kann aber auch jede andere Röntgenanordnung umfassen, bei der der Detektor mit einem Streustrahlenraster versehen ist und beide Komponenten bezüglich eines zu untersuchenden Objekts schwenkbar oder verfahrbar sind. Bei entsprechenden Bewegungen treten in der Regel stets wenn auch nur minimale Dejustierungen des Detektors gegenüber dem idealen Strahlengang auf. Diese mehr oder weniger großen Dejustierungen wirken sich auf die Sichtbarkeit des Streustrahlenrasters auf dem gewonnenen Röntgenbild aus.

Bei der Positioniereinrichtung handelt es sich vorzugsweise um einen C-Bogen oder um Roboterarme. An dem einen Ende des C-Bogens befindet sich üblicherweise die Röntgenröhre und an dem anderen Ende des C-Bogens der Detektor mit Streustrahlenraster. Da ein derartiger C-Bogen mit Röntgenkomponenten durchaus mehrere 100 Kilo wiegen kann, ist unmittelbar einsichtig, dass bei Bewegungen des C-Bogens im dreidimensionalen Raum allein durch das Eigengewicht Verbiegungen und Verwindungen auftreten. Das Maß der Verbiegung beziehungsweise Verwindung hängt maßgeblich auch von der Steifigkeit der Positioniereinrichtung beziehungsweise des C-Bogens ab. Insbesondere hängt das Maß der Verbiegung beziehungsweise Verwindung aber von der Ausrichtung der Positioniereinrichtung beziehungsweise des C-Bogens im Raum ab. Steht der C-Bogen beispielsweise senkrecht, so verbiegt er sich unter Umständen weniger als in Fällen, in denen die Positioniereinrichtung beziehungsweise der C-Bogen gekippt wird.

Als Positioniereinrichtung kann ein C-Bogen verwendet werden. Der C-Bogen beinhaltet in diesem Fall auch die Antriebseinheiten, die ihn in die verschiedenen Positionen und Ausrichtungen bringen. Bei der Positioniereinrichtung kann es sich aber auch beispielsweise um einen Roboterarm handeln, der den Detektor entsprechend platziert und/oder ausrichtet.

Grundsätzlich ist das Röntgensystem in der Lage, den Detektor im Raum an verschiedenen Positionen zu positionieren. Beispielsweise kann ein C-Bogen-Röntgengerät den Detektor auf Teilen einer Kugeloberfläche positionieren beziehungsweise platzieren. In jeder dieser Positionen ist der C-Bogen entsprechend um eine oder mehrere Achsen gekippt. In jeder dieser Positionen verbiegt beziehungsweise verwindet sich der C-Bogen beziehungsweise die Positioniereinrichtung entsprechend ihrer Aufhängung, Gewichtsverteilung, Steifigkeit et cetera.

Nun erfolgt in jeder dieser Positionen mindestens eine Röntgenaufnahme. Je nach Verbiegung und/oder Verwindung wird die Rasterstruktur des Streustrahlenrasters unterschiedlich stark auf dem Bild zu erkennen sein. Im Idealfall bei keiner Verbiegung und Verwindung ist das Streustrahlenraster eher als filigrane Struktur zu erkennen. Bei größerer Verbiegung beziehungsweise Verwindung werden die Strukturelemente der Rasterstruktur breiter, da die Rasterschächte nicht mehr parallel zum Strahlengang sind. So ergeben sich also in allen Positionen des Detektors unterschiedliche Abbildungen des Streustrahlenrasters. Wenn also beispielsweise das C-Bogen-Röntgengerät zwei Rotationsfreiheitsgrade besitzt und der erste Rotationsfreiheitsgrad eine Drehbarkeit um 360 Grad erlaubt, können in einem Raster von beispielsweise fünf Grad, zehn Grad oder dergleichen Aufnahmen getätigt werden. Am Ende dieser 360 Grad wird der C-Bogen im zweiten Freiheitsgrad um ein Rasterelement (beispielsweise ebenfalls fünf Grad oder zehn Grad) weitergedreht. Anschließend wird wieder im ersten Freiheitsgrad die Rasterung durchfahren. So lässt sich beispielsweise eine Kugeloberfläche abtasten.

Mit den zahlreichen Röntgenaufnahmen liegt ein Datensatz vor, der die Auswirkungen der geometrischen Änderungen des Röntgensystems in Bezug auf die Abbildung des Streustrahlenrasters zeigt. Aus diesem Datensatz können nun die Artefakte des Streustrahlenrasters in jeder Position für die spezielle Funktion gelernt werden. Die jeweils erzeugten Artefakte entsprechen Abbildungskomponenten, die nicht oder nicht nur durch senkrechte Projektion des Streustrahlenrasters auf die Bildebene entstehen. Dabei kann es sich beispielsweise um Verbreiterungen oder Verwischungen der Gitterstruktur handeln.

Das Lernen bzw. Trainieren der Artefakte muss nicht zwangsläufig in Bezug zu den jeweiligen Positionen stehen. Vielmehr kann es ausreichen, dass das System lernt, dass es sich bei einer abgebildeten Struktur eben genau um eine Rasterstruktur eines Streustrahlenrasters handelt. Das System erkennt dann nicht nur das Streustrahlenraster in der idealen Ausrichtung parallel zum Strahlengang, sondern auch das Streustrahlenraster, wenn es in eine oder mehrere Richtungen relativ zum Strahlengang verkippt ist.

In einer vorteilhaften Ausgestaltung des erfindungsgemäßen Verfahrens werden bei jedem Positionieren des Detektors jeweilige Anlagengeometriedaten des Röntgensystems bereitgestellt, die als Eingangsgrößen für das automatische Lernen dienen. Wie oben bereits erwähnt wurde, müssen diese Anlagengeometriedaten nicht in jedem Fall zur Verfügung stehen. Es ist jedoch von Vorteil, wenn sie zur Verfügung stehen und zusammen mit dem jeweiligen Bild auch mitgelernt werden können, sodass die jeweilige Klassifizierung für die Funktion des Röntgensystems sicherer durchgeführt werden kann.

Des Weiteren kann vorgesehen sein, dass jede der verschiedenen Positionen des Detektors für das automatische Lernen mehrfach von der Positioniereinrichtung angefahren wird. Durch das mehrfache Anfahren ein und derselben Position entsteht der Vorteil, dass dem variierenden Verhalten des Röntgensystems Rechnung getragen werden kann. Es hat sich nämlich gezeigt, dass eine erste Fahrt zu einem Rasterpunkt häufig etwas andere Artefakte hervorruft wie eine zweite Fahrt zu demselben Rasterpunkt. Dies bedeutet, dass beim mehrfachen Anfahren dieses Rasterpunkts und dem entsprechenden mehrfachen Aufnehmen korrespondierender Röntgenbilder unterschiedliche, durch das Streustrahlenraster hervorgerufene, Artefakte erzeugt beziehungsweise abgebildet werden. Durch mehrere Aufnahmen kann entsprechend eine Mittelung der Artefakte durchgeführt werden. Dadurch erhöht sich die Sicherheit, mit der die jeweiligen Artefakte korrekt erkannt werden können.

Speziell kann vorgesehen sein, dass jede Position beziehungsweise jeder Rasterpunkt von einer Vielzahl an Rasterpunkten aus verschiedenen Richtungen angefahren wird. Die Verbiegung und/oder Verwindung hängt nämlich auch von der Historie der vorhergehenden Bewegungen der Positioniereinrichtung beziehungsweise des C-Bogens ab. Das System geht nämlich bei jeder Bewegung nicht vollständig in den Ausgangszustand (unverbogener Zustand) zurück. Die Verbiegungen beziehungsweise Verwindungen sind also nicht vollkommen reversibel und hängen vielmehr von der Startposition ab, aus der sich der Detektor in die neue Position bewegt hat. Dabei spielen auch Schwingungen des Systems beziehungsweise Detektors einschließlich Streustrahlenraster eine Rolle. So hängen die Schwingungen typischerweise vom jeweiligen Antrieb ab. Insbesondere kann beispielsweise ein Antrieb um eine Achse andere Schwingungen hervorrufen wie ein Antrieb um eine andere Achse. Sind die Schwingungen nicht ausreichend abgeklungen, so wirken sie sich entsprechend unterschiedlich auf die Artefakte aus.

In einer weiteren vorteilhaften Ausgestaltung ist die Vielzahl an verschiedenen Positionen des Detektors gleichmäßig über einen gesamten systembedingten Bewegungsraum des Röntgengeräts verteilt angeordnet. Es sind also Rasterpunkte gleichmäßig in demjenigen Raum verteilt, den der Detektor einnehmen kann. Dabei kann der Detektor idealisiert als Punkt angenommen werden.

Darüber hinaus kann vorgesehen sein, dass das automatische Lernen zusätzlich zum ersten Lernschritt einen zweiten Lernschritt umfasst, der gleich dem ersten Lernschritt ist, wobei jedoch das Streustrahlenraster von dem Detektor abgenommen ist. In dem ersten Lernschritt werden also die Artefakte des tatsächlich präsenten Streustrahlenrasters gelernt, während in dem zweiten Lernschritt gelernt wird, wie die Bilder ohne Streustrahlenraster aussehen. Auf diese Weise kann das System die Effekte des Streustrahlenrasters besser erkennen, sodass sie letztlich besser eliminiert werden können.

Entsprechend einer Weiterbildung des Verfahrens wird bei dem Aufnehmen der Röntgenaufnahmen ein Phantom, das (möglichst) keine Streustrahlung erzeugt, im Strahlengang platziert. Das Phantom entspricht einem künstlichen Objekt, das von der Röntgenstrahlung durchleuchtet wird. Die aufgenommenen Röntgenbilder enthalten also nicht nur eine Abbildung des Streustrahlenrasters, sondern auch eine Abbildung des Phantoms. Durch das automatische Lernen kann sicherer zwischen den Abbildungskomponenten des Phantoms und den Abbildungskomponenten des Streustrahlenrasters unterschieden werden. Insbesondere ist der Lerneffekt deshalb höher, weil die Röntgenaufnahmen aus unterschiedlichen Blickwinkeln getätigt werden.

Entsprechend einem weiteren Aspekt der vorliegenden Erfindung werden virtuelle Trainingsdaten durch Simulation gewonnen und zusätzlich zu den Röntgenaufnahmen zum automatischen Lernen verwendet. Dies hat den Vorteil, dass zusätzliche Trainingsdaten rasch und kostengünstig bereitgestellt werden können, um das Signal-zu-Rausch-Verhältnis zu verbessern. Eventuell müssen nur in bestimmten Bereichen des Bewegungsraums tatsächliche Röntgenbilder für das automatische Lernen beziehungsweise Trainieren gewonnen werden, und in anderen Raumteilen können aufgrund von Symmetrien virtuelle Trainingsdaten verwendet werden. Gegebenenfalls können auch stärkere Vibrationen simuliert werden, die sich bei der Alterung der Geräte ergeben können, und die unter Umständen Verbreiterungen der Artefakte hervorrufen. Gegebenenfalls können virtuelle Trainingsdaten auch durch Überlagerungen von bereits gewonnenen Röntgenbildern gewonnen werden.

Vorzugsweise ist auch vorgesehen, dass von einem Objekt eine Objekt-Röntgenaufnahme gewonnen wird und Artefakte des Streustrahlenrasters in dieser Objekt-Röntgenaufnahme mittels der gelernten Artefakte reduziert oder eliminiert werden. Die Ausgangsdaten dieses automatischen beziehungsweise maschinenbasierten Lernens oder Trainierens werden also dazu genutzt, die Artefakte bei einer tatsächlichen Röntgenaufnahme teilweise oder vollständig zu reduzieren. Als Resultat ergibt sich dann eine korrigierte Objekt-Röntgenaufnahme im Wesentlichen ohne die Artefakte des Streustrahlenrasters. In einem vereinfachten Fall wird beispielsweise in einer Objekt-Röntgenaufnahme die Rasterstruktur des Streustrahlenrasters (positionsspezifisch) erkannt und von der Objekt-Röntgenaufnahme softwaretechnisch subtrahiert. Somit lässt sich eine Röntgenaufnahme des Objekts gewinnen, die keine positionsspezifischen Artefakte des Streustrahlenrasters besitzt.

Erfindungsgemäß kann in einer Anwendung der trainierten Funktion auch bereitgestellt werden ein Verfahren zum Betreiben eines Röntgensystems, das eine Positioniereinrichtung, insbesondere einen C-Bogen, mit einem Detektor und im Strahlengang vor dem Detektor ein Streustrahlenraster aufweist, durch
- Positionieren des Detektors an einer Position, wobei sich die Positioniereinrichtung verbiegt und/oder verwindet,
- Aufnehmen einer Röntgenaufnahme in der Position und
- Korrigieren von bei dem Verbiegen und/oder Verwinden der Positioniereinrichtung entstehenden Artefakten, die durch das Streustrahlenraster hervorgerufen werden, in der Röntgenaufnahme mittels entsprechend einem der oben geschilderten Verfahren gelernter Artefakte beziehungsweise Funktion.

Entsprechend einem weiteren Aspekt der vorliegenden Erfindung ist vorgesehen ein computerimplementiertes Verfahren zum Erzeugen von Objektbilddaten, aufweisend die Schritte:
- Empfangen von einer Objekt-Röntgenaufnahme
- Anwenden einer Funktion gemäß dem oben genannten Verfahren auf die Objekt-Röntgenaufnahme zum Erzeugen einer korrigierten Objekt-Röntgenaufnahme, und
- Bereitstellen der korrigierten Objekt-Röntgenaufnahme als die Objektbilddaten.

Das erfindungsgemäße Verfahren kann also im Rahmen eines computerimplementierten Verfahrens realisiert werden, bei dem die Objektröntgenaufnahme entsprechend der gelernten Funktion um die Artefakte korrigiert wird.

Entsprechend einem weiteren Aspekt der vorliegenden Erfindung wird bereitgestellt ein Röntgensystem mit
- einer Positioniereinrichtung und
- einer Aufnahmeeinrichtung mit einem Detektor und einem im Strahlengang vor dem Detektor befindlichen Streustrahlenraster, wobei
- der Detektor und das Streustrahlenraster an der Positioniereinrichtung angebracht ist, wobei
- mit der Positioniereinrichtung der Detektor an einer Vielzahl von verschiedenen Positionen, in denen sich der die Positioniereinrichtung positionsabhängig verbiegt und/oder verwindet, positionierbar ist,
- mit der Aufnahmeeinrichtung jeweils mindestens eine Röntgenaufnahme in jeder der Positionen erzeugbar ist und
- das Röntgensystem mit einer Recheneinrichtung zum automatischen Lernen von durch das Streustrahlenraster erzeugten Artefakten aus allen Röntgenaufnahmen in einem ersten Lernschritt ausgestattet ist.

Bei dem Röntgensystem handelt es sich beispielsweise um ein C-Bogen-Röntgensystem oder ein anderes System, bei dem der Detektor unterschiedlich positioniert werden kann. Hierzu dient die Positioniereinrichtung, an der der Detektor und das Streustrahlenraster befestigt sind. Die Positioniereinrichtung wurde oben im Zusammenhang mit dem erfindungsgemäßen Verfahren bereits näher erläutert. Die Aufnahmeeinrichtung des Röntgensystems umfasst in selbstverständlicher Weise neben dem Detektor und dem Streustrahlenraster auch eine entsprechende Röntgenquelle. Mit der Recheneinrichtung, die beispielsweise einen oder mehrere Prozessoren beinhalten kann, wird das automatische Lernen durchgeführt.

Das automatische Lernen beziehungsweise Maschinenlernen kann durch ein überwachtes Training, ein halbüberwachtes Training, bestärkendes Lernen und/oder aktives Lernen beinhalten. Darüber hinaus kann das automatische Lernen auch ein Repräsentationslernen (englisch: "feature learning") beinhalten. Speziell können die Parameter der trainierten Funktionen iterativ in mehreren Trainingsschritten adaptiert werden.

Insbesondere kann die trainierte Funktion ein neuronales Netzwerk, eine Support-Vektor-Maschine, einen Entscheidungsbaum und/oder ein Bayessches Netzwerk aufweisen. Darüber hinaus kann die trainierte Funktion auf einem k-Means-Algorithmus, einem Q-Lern-Algorithmus, einem genetischen Algorithmus und/oder auf Assoziationsregeln basieren. Speziell kann ein neuronales Netzwerk ein tiefes neuronales Netzwerk, ein faltendes neuronales Netzwerk (englisch: "convolutional neural network") oder ein faltendes, tiefes neuronales Netzwerk sein. Ferner kann das neuronale Netzwerk ein gegnerisches Netzwerk (englisch: "adversarial network"), ein tiefes gegnerisches Netzwerk und/oder ein erzeugendes gegnerisches Netzwerk (englisch: "generative adversarial network") sein.

Die oben im Zusammenhang mit dem erfindungsgemäßen Verfahren geschilderten Vorteile und Variationsmöglichkeiten gelten sinngemäß auch für das erfindungsgemäße Röntgensystem und umgekehrt.

In dem vorliegenden Dokument werden erfindungsgemäße Lösungen mit Bezug auf Verfahren zum Betreiben eines Röntgensystems sowie entsprechende Röntgensysteme, aber auch mit Bezug auf Verfahren zum Trainieren eines Röntgensystems beziehungsweise entsprechende Röntgensysteme beschrieben. Merkmale, Vorteile und alternative Ausführungsformen gelten wechselweise für sowohl die entsprechenden Verfahren als auch die entsprechenden Röntgensysteme. Mit anderen Worten, die Ansprüche für Verfahren und Systeme zum Trainieren können mit Merkmalen weitergebildet werden, die im Zusammenhang mit den Verfahren und Systemen für die Anwendung beschrieben oder beansprucht sind, und umgekehrt.

Im Allgemeinen ahmt die trainierte Funktion (basierend auf den gelernten Artefakten) kognitive Funktionen nach. Insbesondere ist die trainierte Funktion in der Lage, sich auf neue Umstände einzustellen und entsprechende Muster zu detektieren und zu extrapolieren.

Die vorliegende Erfindung wird nun anhand der beigefügten Zeichnungen näher erläutert, in denen zeigen:
- FIG 1: einen schematischen Verfahrensablauf; und
- FIG 2: eine skizzenhafte Darstellung eines Röntgensystems.

Die nachfolgend näher geschilderten Ausführungsbeispiele stellen bevorzugte Ausführungsformen der vorliegenden Erfindung dar.

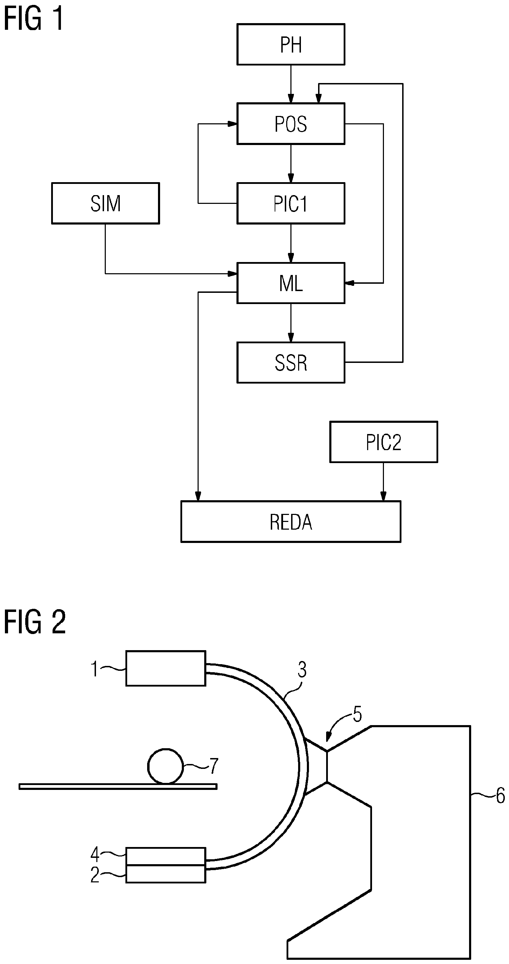

Das Beispiel von FIG 1 zeigt ein schematisches Ablaufdiagramm eines erfindungsgemäßen Verfahrens zum Betreiben eines Röntgensystems. In einem optionalen Schritt PH wird ein Phantom im Strahlengang des Röntgensystems und insbesondere des C-Bogen-Röntgensystems platziert. Bei dem Phantom kann es sich um ein Objekt handeln, das ähnliche Formen und Strukturen besitzt wie eine natürliche anatomische Struktur. Vorzugsweise ruft das Phantom im Strahlengang keine oder nur sehr wenig Streustrahlung hervor, wenn das Phantom von Röntgenstrahlung durchleuchtet wird.

In einem Positionierschritt POS wird ein Detektor des Röntgensystems an einer bestimmten Position im Raum positioniert. Vor dem Detektor befindet sich im Strahlengang ein Streustrahlenraster. Entsprechend dem Strahlengang gegenüber dem Detektor befindet sich eine Röntgenquelle. Bei einem C-Bogen-System wird die Röntgenquelle in bekannter Weise mit dem Detektor bewegt, da beide Komponenten an den C-Bogen-Armen befestigt sind.

In einem Aufnahmeschritt PIC1 wird eine Röntgenaufnahme gemacht. Anschließend wird der Detektor neu positioniert, sodass das Verfahren zu dem Positionierschritt POS zurückspringt. Dadurch wird der Detektor im Raum neu ausgerichtet und platziert. Anschließend wird in dieser neuen Position des Detektors wieder eine Röntgenaufnahme gemacht. Diese Schritte POS und PIC1 werden so oft wiederholt, bis alle Rasterpunkte in einem vorgegebenen Bewegungsraum des Detektors einmal oder mehrmals eingenommen wurden. Vorzugsweise ist der gesamte Bewegungsraum des Detektors beziehungsweise Aufnahmesystems gleichmäßig mit Rasterpunkten versehen. Beispielsweise können die Rasterpunkte auf einem gleichmäßigen Gitter liegen, das über den gesamten Bewegungsraum aufgespannt ist. Die Rasterpunkte können beispielsweise dadurch definiert sein, dass sich das Aufnahmesystem jeweils um konstante Winkelbeträge diskret um eine erste Achse und in gleicher Weise diskret um eine zweite senkrechte Achse dazu bewegt. So kann beispielsweise das System von einem Rasterpunkt zum nächsten um fünf oder zehn Grad (oder einen anderen Gradwert) bewegt werden.

In jeder Position beziehungsweise Ausrichtung des Detektors einschließlich des Streustrahlenrasters verbindet beziehungsweise verbiegt sich das System beziehungsweise die Positioniereinrichtung des Röntgensystems auf charakteristische Weise. Auch wenn derartige Verbiegungen beziehungsweise Verwindungen gering und kaum wahrnehmbar sind, spielen sie für die Abbildung des Streustrahlenrasters auf dem Detektor unter Umständen maßgebliche Rolle. Da das Ausmaß der Verbiegungen und Verwindungen von der jeweiligen Positionierung des Detektors beziehungsweise des gesamten Abbildungssystems abhängt, hängen auch die bei der Bildgebung entstehenden Artefakte entsprechend von der Detektorpositionierung ab.

Je nach System kann die Positionierung eines Detektors auf unterschiedliche Weise erfolgen. Bei C-Bogen-Röntgensystemen sind in grober Näherung feste Bewegungsbahnen auf einer oder mehreren Kugeloberflächen vorgegeben. Bei roboterbasierten Röntgensystemen wird ein Detektor beispielsweise um eine erste Achse linear verschoben und um eine oder mehrere Achsen geschwenkt. In diesem Fall kann beispielsweise die Detektorposition vollkommen unabhängig von der Position der Röntgenquelle eingestellt werden. Roboterbasierte Röntgensysteme können am Boden oder an der Decke montiert sein. Unabhängig davon kommt es bei allen Positionierungen und Ausrichtungen des Detektors zu intrinsischen Verbiegungen beziehungsweise Verwindungen des Systems, z.B. des Roboterarms.

Diese systembedingten Verbiegungen und Verwindungen sind häufig nicht oder nur sehr schwer reproduzierbar. In einer ersten Position verbiegt sich das System auf eine erste Weise. Falls das System in eine zweite Position gebracht wird, verbiegt beziehungsweise verwindet es sich auf eine zweite Weise, die von der ersten Weise abhängt. Dies liegt daran, dass das System bei einem Positionswechsel nicht immer in die gleiche Ausgangsposition zurückkehrt. Startend von einer dritten Position ergibt sich in der zweiten Position also eine andere Verbiegung beziehungsweise Verwindung als bei einem Start von der ersten Position.

In einem automatischen Lernschritt beziehungsweise Maschinenlernschritt ML werden nun die durch das Streustrahlenraster hervorgerufenen Artefakte aus der Vielzahl der aufgenommenen Röntgenaufnahmen für eine Funktion des Röntgensystems gelernt. Dabei kann es unter Umständen günstig sein, die aus dem Positionierschritt POS gegebenenfalls bereitgestellten Positionsdaten mit zu berücksichtigen. Das System lernt also die durch das Streustrahlenraster hervorgerufenen Artefakte in vielen beziehungsweise allen möglichen Positionen. Gegebenenfalls werden bei dem Lernen auch die Bewegungshistorie und insbesondere die Position des Detektors vor der jeweils aktuellen Detektorposition mitgelernt. Es werden also beispielsweise eine aktuelle Röntgenaufnahme, eine aktuelle Position und eine vorhergehende Position in einem Lernschritt verknüpft.

Um die Menge der Trainingsdaten für das Training des Systems zu erhöhen, können zusätzliche virtuelle Trainingsbilder erzeugt werden. Derartige virtuelle Trainingsbilder können durch eine Simulation in einem Simulationsschritt SIM gewonnen werden. So können diese virtuellen Trainingsbilder durch ein virtuelles Streustrahlenraster mittels einer entsprechenden Simulation gewonnen werden. Hierdurch lässt sich die Basis der Trainingsdaten vergrößern und somit die Qualität des Lernens verbessern.

In einem weiteren Verfahrensschritt der Streustrahlenrasterentfernung SSR kann das Streustrahlenraster aus dem Strahlengang zwischen Röntgenquelle und Röntgendetektor herausgenommen werden. Hierdurch können Röntgenaufnahmen gewonnen werden, die keine Artefakte besitzen, welche durch das Streustrahlenraster entstehen. Gegebenenfalls befinden sich auf den Röntgenaufnahmen nur noch Durchleuchtungsbilder des Phantoms. Anhand dieser Bilder kann das lernende System den Unterschied zwischen den Strukturen des Phantoms und den Strukturen des Streustrahlenrasters lernen. Nach Herausnahme des Streustrahlenrasters wird das Verfahren also bei dem Positionierschritt POS weitergeführt und es werden die gleichen Röntgenaufnahmen gewonnen wie zuvor mit dem Streustrahlenraster.

Das Maschinenlernen ML erfolgt hier also mit Bildern, die das Streustrahlenraste einschließlich Artefakte zeigen, mit Bildern, die ohne Streustrahlenraster aufgenommen wurden und gegebenenfalls auch mit virtuellen Trainingsbildern aus der Simulation SIM. Das so gelernte System bzw. die so gelernte Funktion kann nun im praktischen Betrieb angewandt werden. Dazu werden in einem weiteren Aufnahmeschritt PIC2 Objekt-Röntgenaufnahmen eines zu untersuchenden Objekts, z.B. eines Teils eines menschlichen Körpers, gewonnen. Die Aufnahme beziehungsweise die Aufnahmen entstanden mit dem Streustrahlenraster. In einem Bildverarbeitungsschritt REBA werden aus den Objekt-Röntgenaufnahmen die Rasterstrukturen des Streustrahlenrasters einschließlich der positionsabhängigen Artefakte, die ebenfalls von dem Streustrahlenraster herrühren, durch die gelernte Funktion reduziert beziehungsweise eliminiert. Gegebenenfalls werden hierfür auch die aktuellen Positionsdaten der Detektorposition beziehungsweise der Position der Aufnahmeeinrichtung verwendet. Diese Positionsdaten können natürlich auch wieder Rechnungsinformationen über die Ausrichtung des Detektors beziehungsweise der Aufnahmeeinrichtung beinhalten.

FIG 2 zeigt schematisch ein Ausführungsbeispiel eines Röntgensystems. Im vorliegenden Fall handelt es sich um ein C-Bogen-Röntgensystem. Es besitzt eine Röntgenquelle 1 und einen Detektor 2 an den gegenüberliegenden Armen eines C-Bogens 3. In dem Strahlengang zwischen der Röntgenquelle 1 und dem Detektor 2 befindet sich unmittelbar an dem Detektor 2 ein Streustrahlenraster 4. Die Röntgenquelle 1 zusammen mit dem Detektor 2 und gegebenenfalls dem eingesetzten Streustrahlenraster 4 bilden eine Aufnahmeeinrichtung. Der C-Bogen 3 gegebenenfalls zusammen mit einem Dreh- oder Schwenkgelenk 5 sowie entsprechenden Antrieben für den C-Bogen 3 bilden eine Positioniereinrichtung für die Aufnahmeeinrichtung 1, 2, 4. In einem Gerätekörper 6 kann eine entsprechende Steuerung und Bildverarbeitung beziehungsweise eine entsprechende Recheneinrichtung untergebracht sein.

Die Röntgenquelle 1 und der Detektor 2 mit abnehmbarem Streustrahlenraster 4 können alternativ auch von anderen Halteeinrichtungen, wie etwa Roboterarmen, gehalten und positioniert werden. Auch hier sei nochmals betont, dass das Positionieren auch ein Ausrichten beinhalten kann.

Im Strahlengang zwischen der Röntgenquelle 1 und dem Detektor 2 befindet sich gegebenenfalls ein Phantom 7 oder das tatsächlich zu untersuchende Objekt. Das Objekt erzeugt in der Regel eine Streustrahlung, die von dem Streustrahlenraster 4 bei idealer Ausrichtung von Röntgenquelle 1 und Detektor 2 größtenteils eliminiert wird, da das Streustrahlenraster ideal zum Strahlengang ausgerichtet ist. Auf dem Detektor ergibt sich dann ein scharfes Bild des Streustrahlenrasters 4, welches leicht durch einfache Subtraktion eliminiert werden kann. Falls jedoch der C-Bogen 3 gekippt oder gedreht wird, kann dies dazu führen, dass sich der eine Arm des C-Bogens 3 geringfügig anders verbiegt beziehungsweise verwindet als der andere Arm des C-Bogens 3. In diesem Fall entstehen an der Rasterstruktur im Röntgenbild zusätzliche Artefakte. Diese lassen sich mithilfe des gelernten Systems, das die Artefakte in allen möglichen Positionen des Detektors 2 beziehungsweise der Aufnahmeeinrichtung "kennt", eliminieren. Dabei kann das gelernte System auch die eigentliche Rasterstruktur aus den Röntgenaufnahmen entfernen.

In einem konkreten Beispiel erfolgt eine Anwendung eines lernbasierten Verfahrens auf der Basis von sogenannten "Kalibrier-Runs" in der Fertigung oder beim Anwender, um die Rasterstrukturen zu lernen und gegebenenfalls einschließlich der Artefakte zu eliminieren.

Das Training des Systems erfolgt beispielsweise in zwei Lernschritten A und B. Der Lernschritt A gestaltet sich wie folgt:
a) Das Streustrahlenraster ist eingesetzt.
b) Der gesamte Raum der möglichen Bewegungstrajektorien wird ausreichend genau abgetastet (z.B. Schrittweite von fünf oder zehn Grad).
c) Dabei wird für jeden Abtastpunkt Strahlung ausgelöst und das Ergebnisbild gespeichert.
d) Zusätzlich kann die Anlagengeometrie zu diesem Punkt mitgespeichert werden.
e) Als Option kann noch ein anatomisches Phantom im Strahlengang platziert werden.

Der Lernschritt B gestaltet sie wie folgt:
f) Das Raster ist herausgenommen.
g) Der gesamte Raum der möglichen Bewegungstrajektorien wird ausreichend genau abgetastet (z.B. Schrittweite von fünf oder zehn Grad).
h) Dabei wird für jeden Abtastpunkt Strahlung ausgelöst und das Ergebnisbild gespeichert.
i) Zusätzlich kann die Anlagengeometrie zu diesem Punkt mitgespeichert werden.
j) Als Option kann noch ein anatomisches Phantom im Strahlengang platziert werden.

Die Daten aus Lernschritt A und B werden für das lernbasierte Verfahren genutzt, um es zu trainieren. Gegebenenfalls erfolgt das Training durch ein überwachtes Training, ein halbüberwachtes Training, ein unüberwachtes Training oder dergleichen. Hierzu kann ein neuronales Netz, eine Sapport-Vektor-Maschine oder dergleichen verwendet werden. In der Anwendung wird dann das trainierte Netzwerk beziehungsweise die trainierte Funktion verwendet, um die Rasterstruktur einschließlich Artefakte zu eliminieren.

Durch die Anwendung eines lernbasierten Verfahrens ist für die Korrektur eine genaue Kenntnis der Abbildung nicht notwendig. Jede Veränderung muss nicht neu analytisch erfasst werden, sondern kann durch einen neuen Kalibrier-Run gelöst werden.

## Patentansprüche

1. Verfahren zum Trainieren einer Funktion eines Röntgensystems, das eine Positioniereinrichtung (3, 5), insbesondere einen C-Bogen (3), mit einem Detektor (2) und im Strahlengang vor dem Detektor (2) ein Streustrahlenraster (4) aufweist, **gekennzeichnet durch**
- Positionieren (POS) des Detektors (2) an einer Vielzahl an verschiedenen Positionen, wobei sich die Positioniereinrichtung (3, 5) verbiegt und/oder verwindet,
- Aufnehmen (PIC1) jeweils mindestens einer Röntgenaufnahme in jeder der Positionen und
- automatisches Lernen (ML) von durch das Streustrahlenraster (4) erzeugter Artefakte aus allen Röntgenaufnahmen für die Funktion in einem ersten Lernschritt.

2. Verfahren nach Anspruch 1, wobei bei jedem Positionieren (POS) des Detektors (2) jeweilige Anlagengeometriedaten des Röntgensystems bereitgestellt werden, die als Eingangsgrößen für das automatische Lernen (ML) dienen.

3. Verfahren nach Anspruch 1 oder 2, wobei jede der verschiedenen Positionen des Detektors (2) für das automatische Lernen (ML) mehrfach von der Positioniereinrichtung (3, 5) angefahren wird.

4. Verfahren nach Anspruch 3, wobei jede Position aus verschiedenen Richtungen angefahren wird.

5. Verfahren nach einem der vorhergehenden Ansprüche, wobei die Vielzahl an verschiedenen Positionen des Detektors (2) gleichmäßig über einen gesamten systembedingten Bewegungsraum des Röntgensystems verteilt angeordnet ist.

6. Verfahren nach einem der vorhergehenden Ansprüche, wobei das automatische Lernen (ML) zusätzlich zum ersten Lernschritt einen zweiten Lernschritt umfasst, der gleich dem ersten Lernschritt ist, wobei jedoch das Streustrahlenraster (4) von dem Detektor (2) abgenommen und aus dem Strahlengang entfernt (SSR) ist.

7. Verfahren nach einem der vorhergehenden Ansprüche, wobei bei dem Aufnehmen der Röntgenaufnahmen ein Phantom (7), das keine Streustrahlung erzeugt, im Strahlengang platziert ist.

8. Verfahren nach einem der vorhergehenden Ansprüche, wobei durch Simulation (SIM) virtuelle Trainingsdaten gewonnen werden und zusätzlich zu den Röntgenaufnahmen zum automatischen Lernen (ML) verwendet werden.

9. Verfahren nach einem der vorhergehenden Ansprüche, wobei von einem Objekt eine Objekt-Röntgenaufnahme gewonnen wird (PC2) und Artefakte des Streustrahlenrasters (4) in dieser Objekt-Röntgenaufnahme mittels der trainierten Funktion reduziert oder eliminiert werden.

10. Verfahren zum Betreiben eines Röntgensystems, das eine Positioniereinrichtung (3, 5), insbesondere einen C-Bogen (3), mit einem Detektor (2) und im Strahlengang vor dem Detektor (2) ein Streustrahlenraster (4) aufweist, **gekennzeichnet durch**
- Positionieren (POS) des Detektors (2) an einer Position, wobei sich die Positioniereinrichtung (3, 5) verbiegt und/oder verwindet,
- Aufnehmen (PIC1) einer Röntgenaufnahme in der Position und
- Korrigieren (REDA) von bei dem Verbiegen und/oder Verwinden der Positioniereinrichtung (3, 5) entstehenden Artefakten, die durch das Streustrahlenraster (4) hervorgerufen werden, in der Röntgenaufnahme mittels entsprechend einem der Verfahren 1 bis 9 gelernter Artefakte.

11. Computerimplementiertes Verfahren zum Erzeugen von Objektbilddaten, aufweisend die Schritte:
- Empfangen von einer Objekt-Röntgenaufnahme,
- Anwenden einer Funktion gemäß dem Verfahren nach Anspruch 9 auf die Objekt-Röntgenaufnahme zum Erzeugen einer korrigierten Objekt-Röntgenaufnahme und
- Bereitstellen der korrigierten Objekt-Röntgenaufnahme als die Objektbilddaten.

12. Röntgensystem mit
- einer Positioniereinrichtung (3, 5) und
- einer Aufnahmeeinrichtung (1, 2, 4) mit einem Detektor (2) und einem im Strahlengang vor dem Detektor (2) befindlichen Streustrahlenraster (4), wobei
- der Detektor (2) und das Streustrahlenraster (4) an der Positioniereinrichtung (3, 5) angebracht ist,
**dadurch gekennzeichnet, dass**
- mit der Positioniereinrichtung (3, 5) der Detektor (2) an einer Vielzahl von verschiedenen Positionen, in denen sich der die Positioniereinrichtung (3, 5) positionsabhängig verbiegt und/oder verwindet, positionierbar ist,
- mit der Aufnahmeeinrichtung (1, 2, 4) jeweils mindestens eine Röntgenaufnahme in jeder der Positionen erzeugbar ist und
- das Röntgensystem mit einer Recheneinrichtung zum automatischen Lernen von durch das Streustrahlenraster erzeugten Artefakten aus allen Röntgenaufnahmen in einem ersten Lernschritt ausgestattet ist.

13. Röntgensystem nach Anspruch 12, das als C-Bogen-Röntgensystem oder Roboterarm-basiertes Röntgensystem ausgebildet ist.

## Claims

1. Method for training a function of an X-ray system, which has a positioning mechanism (3, 5), in particular a C-arm (3), having a detector (2) and in the beam path in front of the detector (2) has an anti-scatter grid (4),
**characterised by**
- positioning (POS) the detector (2) at a large number of different positions, wherein the positioning mechanism (3, 5) is deflected and/or distorted,
- recording (PIC1) in each case at least one X-ray photograph in each of the positions and
- machine learning (ML) of artifacts generated by the anti-scatter grid (4) from all X-ray photographs for the function in a first learning step.

2. Method according to claim 1, wherein on each positioning (POS) of the detector (2), respective system geometry data of the X-ray system is supplied, which serves as input variables for machine learning (ML).

3. Method according to claim 1 or 2, wherein each of the different positions of the detector (2) for machine learning (ML) is approached multiple times by the positioning mechanism (3, 5).

4. Method according to claim 3, wherein each position is approached from different directions.

5. Method according to one of the preceding claims, wherein the large number of different positions of the detector (2) is arranged uniformly distributed over an entire system-based movement space of the X-ray system.

6. Method according to one of the preceding claims, wherein machine learning (ML), in addition to the first learning step, comprises a second learning step, which is identical to the first learning step, wherein, however, the anti-scatter grid (4) is removed from the detector (2) and removed from the beam path (SSR).

7. Method according to one of the preceding claims, wherein on recording the X-ray photographs, a phantom (7), which does not generate any scatter radiation, is placed in the beam path.

8. Method according to one of the preceding claims, wherein by way of simulation (SIM), virtual training data is obtained and, in addition to the X-ray photographs, is used for machine learning (ML).

9. Method according to one of the preceding claims, wherein an object X-ray photograph is obtained (PC2) from an object and artifacts of the anti-scatter grid (4) are reduced or eliminated in this object X-ray photograph by means of the trained function.

10. Method for operating an X-ray system, which has a positioning mechanism (3, 5), in particular a C-arm (3), having a detector (2) and in the beam path in front of the detector (2) has an anti-scatter grid (4),
**characterised by**
- positioning (POS) the detector (2) at a position, wherein the positioning mechanism (3, 5) is deflected and/or distorted,
- recording (PIC1) an X-ray photograph in the position and
- correcting (REDA) artifacts resulting from deflection and/or distortion of the positioning mechanism (3, 5), which artifacts are caused by the anti-scatter grid (4), in the X-ray photograph by means of artifacts learned according to one of the methods 1 to 9.

11. Computer-implemented method for generating object image data, having the following steps:
- receiving an object X-ray photograph,
- applying a function according to the method according to claim 9 to the object-X-ray photograph for generating a corrected object-X-ray photograph and
- supplying the corrected object X-ray photograph as the object image data.

12. X-ray system, having
- a positioning mechanism (3, 5) and
- a recording device (1, 2, 4) having a detector (2) and an anti-scatter grid (4) located in the beam path in front of the detector (2), wherein
- the detector (2) and the anti-scatter grid (4) is attached to the positioning mechanism (3, 5),
**characterised in that**
- with the positioning mechanism (3, 5), the detector (2) can be positioned at a large number of different positions in which the positioning mechanism (3, 5) is deflected and/or distorted as a function of position,
- with the recording device (1, 2, 4), in each case at least one X-ray photograph can be generated in each of the positions and
- the X-ray system is fitted with an arithmetic device for machine learning of artifacts generated by the anti-scatter grid from all X-ray photographs in a first learning step.

13. X-ray system according to claim 12, which is designed as a C-arm X-ray system or robotic arm-based X-ray system.

## Revendications

1. Procédé d'apprentissage d'une fonction d'un système à rayons X, qui a un dispositif (3, 5) de mise en position, notamment un arceau (3) en C, comprenant un détecteur (2) et une grille (4) d'anti-diffusion devant le détecteur (2) dans le trajet des rayons,
**caractérisé par**
- la mise (POS) en position du détecteur (2) en une pluralité de positions différentes, le dispositif (3, 5) de mise en position se courbant et/ou se tordant,
- l'enregistrement (PIC1) respectivement d'au moins un enregistrement de rayons X dans chacune des positions et
- l'apprentissage (ML) automatique d'artefacts produits par la grille d'anti-diffusion à partir de tous les enregistrements de rayons X pour la fonction dans un premier stade d'apprentissage.

2. Procédé suivant la revendication 1, dans lequel, dans chaque mise (POS) en position de détecteur (2), on met à disposition des données respectives dé géométrie d'installation du système à rayons X, qui servent de grandeurs d'entrée pour l'apprentissage (ML) automatique.

3. Procédé suivant la revendication 1 ou 2, dans lequel on démarre, par le dispositif (3, 5) de mise en position plusieurs fois, chacune des positions différentes du détecteur (2) pour l'apprentissage (ML) automatique.

4. Procédé suivant la revendication 3, dans lequel on démarre chaque position dans des directions différentes.

5. Procédé suivant l'une des revendications précédentes, dans lequel la pluralité de positions différentes du détecteur (2) est disposée en étant répartie de manière uniforme sur un espace de déplacement d'ensemble, dû au système, du système à rayons X.

6. Procédé suivant l'une des revendications précédentes, dans lequel l'apprentissage (ML) automatique comprend, supplémentairement au premier stade d'apprentissage, un deuxième stade d'apprentissage, qui est pareil au premier stade d'apprentissage, dans lequel toutefois la grille (4) d'anti-diffusion est retirée du détecteur (2) et éloignée (SSR) du trajet des rayons.

7. Procédé suivant l'une des revendications précédentes, dans lequel, à l'enregistrement des enregistrements de rayons X, un fantôme (7), qui ne produit pas de rayonnement de diffusion, est placé dans le trajet des rayons.

8. Procédé suivant l'une des revendications précédentes, dans lequel on obtient, par simulation (SIM), des données d'apprentissage virtuelles et on les utile supplémentairement aux enregistrements de rayons X pour l'apprentissage (ML) automatique.

9. Procédé suivant l'une des revendications précédentes, dans lequel on obtient d'un objet un enregistrement de rayons X d'objet (PC2) et on réduit ou on élimine des artefacts de la grille (4) d'anti-diffusion dans cet enregistrement de rayons X d'objet au moyen de la fonction d'apprentissage.

10. Procédé pour faire fonctionner un système à rayons X, qui a un dispositif (3, 5) de mise en position, notamment un arceau (3) en C, comprenant un détecteur (2) et une grille (4) d'anti-diffusion devant le détecteur (2) dans le trajet des rayons,
**caractérisé par**
- la mise (POS) en position du détecteur (2) en une position, le dispositif (3, 5) de mise en position se courbant et/ou se tordant,
- l'enregistrement (PIC1) d'un enregistrement de rayons X dans la position et
- la correction (REDA) d'artefacts créés par la courbure et/ou la torsion du dispositif (3, 5) de mise en position, qui sont provoqués par la grille (4) d'anti-diffusion, dans l'enregistrement de rayons X au moyen d'artefacts ayant subi un apprentissage conformément à l'un des procédés 1 à 9.

11. Procédé, mis en œuvre par ordinateur, de production de données d'images d'objet, comportant les stades
- la réception d'un enregistrement de rayons X d'objet,
- l'application d'une fonction selon le procédé suivant la revendication 9 à l'enregistrement de rayons X-objet pour la production d'un enregistrement de rayons X-objet corrigé et
- la mise à disposition de l'enregistrement de rayons X-objet corrigé comme données d'image d'objet.

12. Système à rayons X comprenant :
- un dispositif (3, 5) de mise en position et
- un dispositif (1, 2, 4) d'enregistrement, comprenant un détecteur (2) et une grille (4) d'anti-diffusion se trouvant devant le détecteur (2) dans le trajet des rayons, dans lequel
- le détecteur (2) et la grille (4) d'anti-diffusion sont montés sur le dispositif (3, 5) de mise en position,
**caractérisé en ce que**
- par le dispositif (3, 5) de mise en position, le détecteur (2) peut être mis en position en une pluralité de positions différentes, dans lesquelles le dispositif (3, 5) de mise en position se courbe et/ou se tord en fonction de la position,
- par le dispositif (1, 2, 4) d'enregistrement, respectivement au moins un enregistrement de rayons X peut être produit dans chacune des positions et
- le système de rayons X est équipé d'un dispositif informatique pour l'apprentissage automatique d'artefacts produits par la grille d'anti-diffusion à partir de tous les enregistrements de rayons X dans un premier stade d'apprentissage.

13. Système à rayons X suivant la revendication 12, qui est constitué sous la forme d'un système à rayons X à arceau en C ou d'un système à rayons X reposant sur un bras de robot.
